# EUROPÄISCHE PATENTANMELDUNG

(11) **EP 3 485 884 A1**
(43) Veröffentlichungstag der Anmeldung: **22.05.2019**
(21) Anmeldenummer: 17202432.5
(22) Anmeldetag: 17.11.2017
(51) Int. Cl.: A61K 31/203, A61K 45/06, A61K 31/07, A61K 31/11, A61K 31/167, A61P 9/14, A61P 9/00, A61P 7/04, A61P 43/00

(54) **VERBINDUNG ZUR ANWENDUNG BEI DER BEHANDLUNG EINER BLUTGEFÄSSERKRANKUNG**

(71) Anmelder: Universität Potsdam, 14469 Potsdam (DE)
(72) Erfinder: SEYFRIED, Salim, 13127 Berlin (DE); OTTEN, Cécile, 13189 Berlin (DE); HÄHN, Kristina, 12524 Berlin (DE)
(74) Vertreter: Müller & Schubert

(57) **Zusammenfassung**

Gegenstand der Erfindung sind Verbindungen zur Anwendung bei der Behandlung einer Blutgefäßerkrankung sowie diese Verbindungen umfassende pharmazeutische Zusammensetzungen und Verfahren zur Behandlung von Blutgefäßerkrankungen.

Hierin beschrieben werden Verbindungen der allgemeinen Formel I 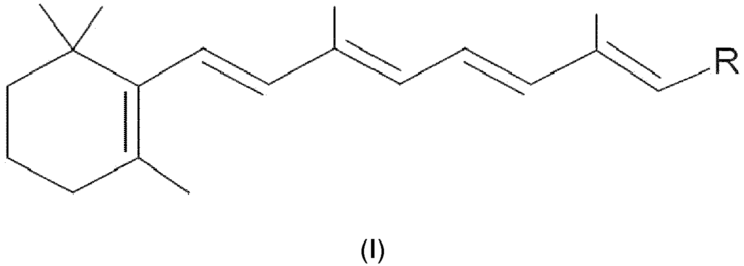 wobei R ausgewählt ist aus der Gruppe bestehend aus -CHO, -COOH, - CH₂OH und 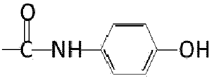 sowie deren Stereoisomere, Enantiomere oder Diastereomere, deren physiologisch annehmbaren Salze sowie Mischungen der genannten Verbindungen zur Anwendung bei der Behandlung einer Blutgefäßerkrankung.

## Beschreibung

Die vorliegende Erfindung betrifft eine Verbindung zur Anwendung bei der Behandlung einer Blutgefäßerkrankung sowie eine diese Verbindung umfassende pharmazeutische Zusammensetzung und ein Verfahren zur Behandlung einer Blutgefäßerkrankung.

Zerebrale kavernöse Malformationen (CCMs) sind Blutgefäßerkrankungen des Menschen, welche zu vaskulären Läsionen im zentralen Nervensystem führen, was wiederum epileptische Anfälle sowie hämorrhagische Schlaganfälle zur Folge haben kann. Kavernome treten sporadisch oder familiär mit autosomal-dominantem Erbgang auf. Über 90 % der erblich bedingten Kavernome liegen Veränderungen im *CCM1-, CCM2-* oder *CCM3*-Gen zugrunde. Das Genprodukt von *CCM1* ist das Protein Krit1 (Krev Interaction Trapped 1), weswegen das *CCM1*-Gen auch häufig als *KRIT1*-Gen bezeichnet wird. PDCD10 (Programmed cell death protein 10) ist das Genprodukt von *CCM3,* weshalb das *CCM3*-Gen auch häufig als *PDCD10*-Gen bezeichnet wird.

Bislang ist die einzige Behandlungsmethode der chirurgische Eingriff zur Entfernung oder Laserverödung von CCMs. Diese Behandlungsmethode ist jedoch mit großen Belastungen für die Betroffenen verbunden. Darüber hinaus ist in vielen Fällen die große Anzahl an Läsionen (hunderte bis tausende von Läsionen in einem Patienten), das Alter des Patienten (*CCM3*-Erkrankungen treten bereits bei Kleinkindern auf), sowie das betroffene Hirnareal (zum Beispiel der Hirnstamm oder tief sitzende Läsionen) für den Chirurgen nicht behandelbar. In diesen Fällen handelt es sich um eine "schicksalhafte Erkrankung" für welche eine pharmakologische Intervention dringend erwünscht ist.

Aufgabe der vorliegenden Erfindung ist es daher, die Nachteile des Standes der Technik zu überwinden und eine Verbindung bereitzustellen, mit der Blutgefäßerkrankungen pharmakologisch behandelt werden können.

Die Aufgabe wird durch die Bereitstellung einer Verbindung zur Anwendung bei der Behandlung einer Blutgefäßerkrankung gemäß den Merkmalen des Hauptanspruchs gelöst. Vorteilhafte Ausgestaltungen der erfindungsgemäßen Verbindung sind in den abhängigen Unteransprüchen gekennzeichnet.

Gegenstand der vorliegenden Erfindung ist eine Verbindung der allgemeinen Formel I 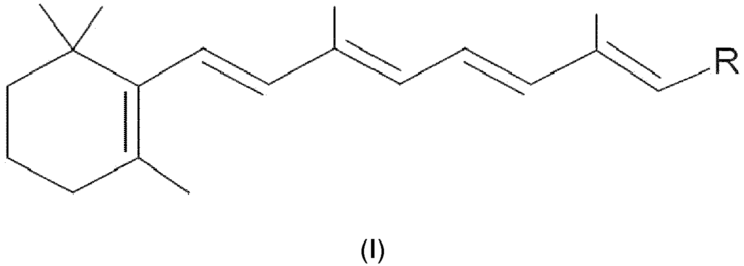 wobei R ausgewählt ist aus der Gruppe bestehend aus -CHO, -COOH, -CH₂OH und 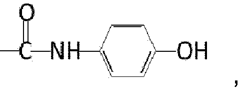 sowie deren Stereoisomere, Enantiomere oder Diastereomere, deren physiologisch annehmbaren Salze sowie Mischungen der genannten Verbindungen zur Anwendung bei der Behandlung einer Blutgefäßerkrankung.

Bevorzugt ist eine erfindungsgemäße Verbindung zur Anwendung bei der Behandlung einer Blutgefäßerkrankung, wobei die Blutgefäßerkrankung ausgewählt ist aus zerebralen kavernösen Malformationen, hämorrhagischen Schlaganfällen, hereditären hämorrhagischen Teleangiektasien oder arterio-venösen Malformationen.

Im Sinne der vorliegenden Erfindung ist die Blutgefäßerkrankung nicht auf die aufgezählten Erkrankungen beschränkt, sondern kann auch ähnliche Erkrankungen umfassen, die verwandte molekulare oder physiologische Ursachen aufweisen.

Insbesondere bevorzugt ist eine erfindungsgemäße Verbindung zur Anwendung bei der Behandlung einer Blutgefäßerkrankung, wobei die Verbindung in einer oral applizierbaren Form vorliegt, wobei die oral applizierbare Form eine Kapsel, eine Tablette, eine Suspension, eine Lösung oder ein Dragee ist oder dass die Verbindung in einer intravenös applizierbaren Form vorliegt, wobei die intravenös applizierbare Form eine Suspension oder eine Lösung ist oder dass die Verbindung in einer subkutan applizierbaren Form vorliegt, wobei die subkutan applizierbare Form eine Suspension, eine Lösung oder ein Pellet ist.

Außerdem ist eine erfindungsgemäße Verbindung zur Anwendung bei der Behandlung einer Blutgefäßerkrankung bevorzugt, wobei die Verabreichung der Verbindung gemäß einem Dosierschema erfolgt, wobei die Dosierung der Verbindung in Abhängigkeit von Alter, Geschlecht und Gewicht eines Patienten, der eine Blutgefäßerkrankung aufweist, sowie von Art und Schwere der Blutgefäßerkrankung festgelegt wird und die Dauer der Behandlung mindestens einen Tag beträgt.

Ein weiterer Gegenstand der vorliegenden Erfindung ist eine pharmazeutische Zusammensetzung zur Anwendung bei der Behandlung einer Blutgefäßerkrankung, umfassend eine erfindungsgemäße Verbindung sowie die physiologisch annehmbaren Salze und Stereoisomere, Enantiomere, Diastereomere oder Mischungen daraus und pharmazeutisch annehmbare Hilfsstoffe und/oder Trägerstoffe in Kombination mit einer oder mehreren Substanzen, ausgewählt aus der Gruppe bestehend aus Indirubin-3-monoxim, Indirubin, Vitamin D, Vitamin D3, Rapamycin, Statinen, Sulindac und seinen Metaboliten, RhoA, Rho-Kinase-Inhibitoren, Gefäßwachstumsfaktor-Inhibitoren, PTC-209, Unc1999, Mek/Erk-Inhibitoren, Propranolol, Propranolol-Hydrochlorid und Antioxidantien.

Im Sinne der vorliegenden Erfindung können die Statine ausgewählt sein aus Simvastatin und Atorvastatin.

Rho-Kinase-Inhibitoren können im Sinne der vorliegenden Erfindung Fasudil sowie BA-1049 und BA2017 sein.

Im Sinne der vorliegenden Erfindung können die Gefäßwachstumsfaktor(VEGF)-Inhibitoren Semaxanib (SU5416) sowie Bevacizumab sein.

Im Sinne der vorliegenden Erfindung ist XMD 8-92 ein Mek/Erk-Inhibitor.

Antioxidantien können im Sinne der vorliegenden Erfindung ausgewählt sein aus der Gruppe bestehend aus N-Acetylcystein, Tempol, Tranilast und Avenantrhramid.

Besonders bevorzugt ist eine erfindungsgemäße pharmazeutische Zusammensetzung zur Anwendung bei der Behandlung einer Blutgefäßerkrankung, wobei die Blutgefäßerkrankung ausgewählt ist aus zerebralen kavernösen Malformationen, hämorrhagischen Schlaganfällen, hereditären hämorrhagischen Teleangiektasien oder arterio-venösen Malformationen.

Insbesondere bevorzugt ist eine pharmazeutische Zusammensetzung zur Anwendung bei der Behandlung einer Blutgefäßerkrankung, wobei die pharmazeutische Zusammensetzung in einer oral applizierbaren Form vorliegt, wobei die oral applizierbare Form eine Kapsel, eine Tablette, eine Suspension, eine Lösung oder ein Dragee ist oder dass die pharmazeutische Zusammensetzung in einer intravenös applizierbaren Form vorliegt, wobei die intravenös applizierbare Form eine Suspension oder eine Lösung ist oder dass die pharmazeutische Zusammensetzung in einer subkutan applizierbaren Form vorliegt, wobei die subkutan applizierbare Form eine Suspension, eine Lösung oder ein Pellet ist.

Außerdem bevorzugt ist eine pharmazeutische Zusammensetzung zur Anwendung bei der Behandlung einer Blutgefäßerkrankung, wobei die Verabreichung der pharmazeutischen Zusammensetzung gemäß einem Dosierschema erfolgt, wobei die Dosierung in Abhängigkeit von Alter, Geschlecht und Gewicht eines Patienten, der eine Blutgefäßerkrankung aufweist, sowie von Art und Schwere der Blutgefäßerkrankung festgelegt wird und die Dauer der Behandlung mindestens einen Tag beträgt.

Ein weiterer Gegenstand der vorliegenden Erfindung ist eine Verfahren zur Behandlung einer Blutgefäßerkrankung, wobei man einem Patienten, der mindestens eine vaskuläre Läsion aufweist oder das Risiko aufweist, eine vaskuläre Läsion zu entwickeln, eine therapeutisch wirksame Menge einer erfindungsgemäßen Verbindung sowie die physiologisch verträglichen Salze und Stereoisomere, Enantiomere, Diastereomere oder Mischungen daraus verabreicht.

Im Sinne der vorliegenden Erfindung ist ein Patient ein Mensch. Die therapeutisch wirksame Menge der Verbindung kann im Sinne der vorliegenden Erfindung von dem Stadium (akute oder chronische Erkrankung) sowie möglicherweise von der Schwere der Erkrankung abhängen (gemessen an der Zahl der CCM Läsionen und dem Auftreten von Blutungen). Insbesondere das Auftreten der Blutungen wird im Feld als der akute Zustand angesehen. Zudem kann eine Dosierung auch prophylaktischer Natur sein.

Beispielsweise benötigt ein Patient mit nur einer CCM Läsion weniger oder eine geringere Dosierung der erfindungsgemäßen Verbindung als ein Patient mit zwei oder mehr CCM Läsionen.

Bevorzugt ist ein erfindungsgemäßes Verfahren, wobei die Blutgefäßerkrankung ausgewählt ist aus zerebralen kavernösen Malformationen, hämorrhagischen Schlaganfällen, hereditären hämorrhagischen Teleangiektasien oder arterio-venösen Malformationen.

Insbesondere bevorzugt ist ein erfindungsgemäßes Verfahren, wobei man vor der Verabreichung sowie nach der Verabreichung der therapeutischen Menge der Verbindung, die Anzahl von vaskulären Läsionen und/oder Größe von mindestens einer vaskulären Läsion sowie das Stadium der Erkrankung erfasst.

Das Stadium der Erkrankung beschreibt im Sinne der vorliegenden Erkrankung, ob es sich um eine akute oder chronische Erkrankung handelt. Darüber hinaus kann die Dosierung auch prophylaktischer Natur sein.

Außerdem bevorzugt ist ein erfindungsgemäßes Verfahren, wobei man die Erfassung der Anzahl von vaskulären Läsionen und/oder die Größe der mindestens einen vaskulären Läsion mittels einer Magnetresonanztomographie durchführt.

Bevorzugt ist ein erfindungsgemäßes Verfahren, wobei die Verbindung oral in Form einer Kapsel, einer Tablette, einer Suspension, einer Lösung oder eines Dragees oder intravenös in Form einer Suspension oder Lösung oder subkutan in Form einer Suspension, einer Lösung oder eines Pellets verabreicht wird.

Insbesondere bevorzugt ist ein erfindungsgemäßes Verfahren, wobei das Verfahren einen Schritt umfasst, in dem ein Patient identifiziert wird, der mindestens eine vaskuläre Läsion aufweist oder das Risiko aufweist, mindestens eine vaskuläre Läsion zu entwickeln.

Weiterhin bevorzugt ist ein erfindungsgemäßes Verfahren, wobei zur Identifizierung eines Patienten, der eine zerebrale kavernöse Malformation aufweist oder das Risiko aufweist eine zerebrale kavernöse Malformation zu entwickeln, eine Mutation in zumindest einem Gen des Patienten identifiziert wird, welches mit der zerebralen kavernösen Malformation assoziiert ist, wobei das Gen ausgewählt ist aus *CCM2, KRIT1* oder *PDCD10.*

Die vorliegende Erfindung wird mit den beigefügten Figuren näher erläutert. Es zeigt:
Fig. 1 Fotos, die einen Überblick über die *CCM*-mutanten Phänotypen im Zebrafischembryo im Vergleich zum Wildtypen geben;
Fig. 2 mikroskopische Aufnahmen, die den Effekt der *CCM2-*Mutation auf die Expression von Aldehyd-Dehydrogenase 1 im Zebrafisch zeigen;
Fig. 3 mikroskopische Aufnahmen des Herzens eines Wildtyp-Zebrafischembryos und einer *CCM2*-Mutante nach der Behandlung mit Retinsäure;
Fig. 4 eine mikroskopische Seitenaufnahmen des Kopfes und Herzens eines Wildtyp-Zebrafischembryos und einer *CCM2*-Mutante nach der Behandlung mit Retinal, Retinol oder Talarozol.

Eine Verbindung der allgemeinen Formel I ist die Retinsäure sowie deren Vorstufen oder Derivate.

Diese Verbindungen haben gegenüber den im Stand der Technik bekannten Substanzen den Vorteil, dass sie spezifisch und signifikant Blutgefäßerkrankungen bei Menschen und Tieren behandeln können. Weiterhin vorteilhaft ist, dass diese Verbindungen einen pharmakologisch unterdrückenden Effekt aufweisen.

Somit ist auch vorteilhaft, dass diese Verbindungen insbesondere für die Behandlung von zerebralen kavernösen Malformationen angewendet werden können. Zerebrale kavernöse Malformationen zeichnen sich insbesondere durch die Bildung von vaskulären Läsionen im zentralen Nervensystem aus. Demnach würde der pharmakologisch unterdrückende Effekt durch Anwendung der Verbindungen gemäß der vorliegenden Erfindung besonders vorteilhaft für die Behandlung von zerebralen kavernösen Malformationen sein.

Die pharmazeutische Zusammensetzung der vorliegenden Erfindung wird mit den üblicherweise verwendeten pharmazeutisch-technischen Hilfsstoffen und Zusatzstoffen entsprechend der gewünschten Applikationsart in einer geeigneten Dosierung in bekannter Weise hergestellt.

Kapseln, welche die erfindungsgemäße pharmazeutische Zusammensetzung enthalten, können beispielsweise hergestellt werden, indem man die vorgesehenen Inhaltsstoffe mit inerten Trägern wie Cellulose, Mikrokristalline Cellulose, Hydroxypropylmethylcellulose, Milchzucker oder Sorbit mischt und in Gelatinekapseln einkapselt.

Entsprechende Tabletten können beispielsweise durch Mischen der vorgesehenen Inhaltsstoffe mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelatine, Gleitmitteln wie Magnesiumstearat oder Talk und/oder Mitteln zur Erzielung eines Depoteffektes wie Carboxypolymethylen, Carboxymethylcellulose, Celluloseacetatphtalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit den vorgesehenen Substanzen der erfindungsgemäßen pharmazeutischen Zusammensetzung können zusätzlich geschmacksverbessernde Mittel wie Saccharin, Cyclamat oder Zucker sowie z.B. Aromastoffe wie Vanillin oder Orangenextrakt enthalten. Sie können außerdem Suspendierhilfsstoffe wie Natriumcarboxymethylcellulose oder Konservierungsstoffe wie p-Hydroxybenzoate enthalten. Als Lösemittel oder Suspendiermittel für Lösungen oder Suspensionen können Wasser, Öle oder andere geeignete Flüssigkeiten verwendet werden.

Weiterhin können die Lösungen auch parenterale Zubereitungen wie Injektionslösungen sein.

Die Herstellung der pharmazeutischen Zusammensetzung folgt den allgemeinen Grundsätzen zur Herstellung von pharmazeutischen Erzeugnissen. Die Herstellung der erfindungsgemäßen pharmazeutischen Herstellung ist an sich bekannt und in den dem Fachmann bekannten Handbücher beschrieben, beispielsweise Hager's Handbuch (5.) 2, 622-1045; List et al., Arzneiformenlehre, Stuttgart: Wiss. Verlagsges. 1985; Sucker et al., Pharmazeutische Technologie, Stuttgart: Thieme 1991; Ullmann's Enzyklopädie (5.) A 19 241-271; Voigt, Pharmazeutische Technologie, Berlin: Ullstein Mosby 1995.

Überraschenderweise haben die Erfinder gefunden, dass die Anwendung einer Verbindung der Formel I die Ausprägung des *CCM*-mutanten Phänotypen im Zebrafischembryo unterdrückt. Dieser Ansatz wird auch am Menschen eine pharmakologische Intervention erlauben, was insbesondere dann vorteilhaft sein wird, wenn kein chirurgischer Eingriff möglich ist. Beispielsweise wenn Läsionen tief im Hirnstamm liegen oder die Läsionen bereits bei Kleinkindern auftreten. Weiterhin vorteilhaft ist, dass eine Reduzierung in der Wachstumsrate und/oder in der Anzahl an CCM Läsionen in einem Patienten auch das Auftreten von mit CCM assoziierten Symptomen, wie zum Beispiel Epilepsie, Hämorrhagie und fokale neurologisch Defizite, reduziert.

Des Weiteren ist die Anwendung einer Verbindung der Formel I auch in der Anwendung zur Behandlung weiterer Blutgefäßerkrankungen vorteilhaft, welche auf ähnlichen molekularen Mechanismen wie die CCM Erkrankung beruhen. Zu diesen Erkrankungen zählen unter anderem hämorrhagische Schlaganfälle, hereditäre hämorrhagische Teleangiektasien oder ateriovenöse Malformationen. Diese Aufzählung stellt jedoch lediglich eine beispielhafte Aufzählung von Blutgefäßerkrankungen dar und keine Beschränkung der vorliegenden Erfindung.

Weiterhin vorteilhaft an der Anwendung einer Verbindung der Formel I zur Behandlung einer Blutgefäßerkrankung ist, dass keine Langzeittherapie notwendig ist, sondern bereits bei kurzzeitigen Behandlungszeiträumen signifikante Verbesserungen der Erkrankung eingeleitet werden.

Die folgenden Verbindungen sind daher, gemäß der vorliegenden Erfindung, besonders bevorzugte Ausführungsformen:

| | |
|---|---|
| Retinsäure | 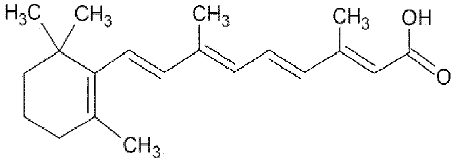 |
| 13-cis-Retinsäure (Isoretinoin) | 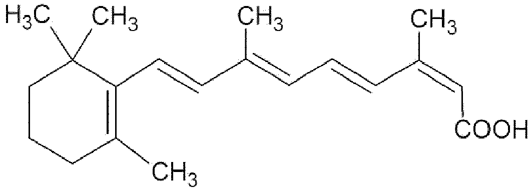 |
| All-trans Retinsäure (Tretinoin) | 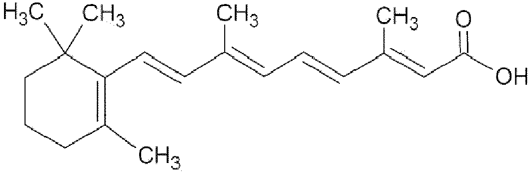 |
| All-trans Retinal | 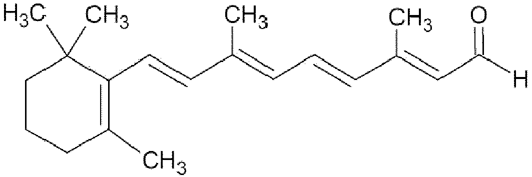 |
| Retinsäure p-hydroxyanilide | 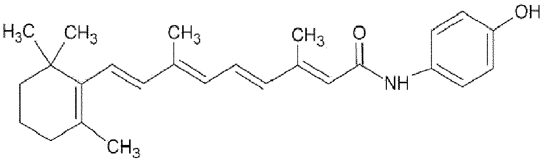 |
| All-trans Retinol | 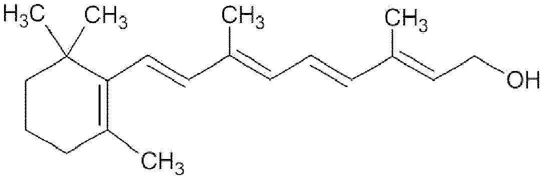 |
| 4-[(E)-2-(5,5,8,8-Tetramethyl-6,7-dihydronaphthalen-2-yl)prop-1-enyl] Benzoesäure (TTNPB) | 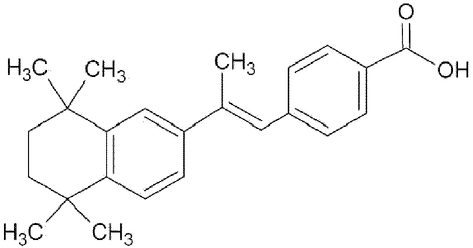 |
| 4'-Octyl-4-biphenylcarbonsäure (AC-55649) | 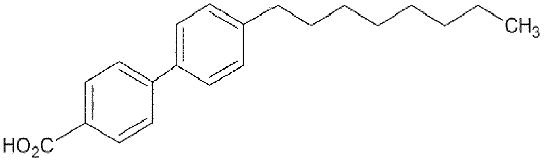 |
| N-(4-(2-Ethyl-1-(1H-1,2,4-triazol-1-<I) butyl) phenyl)-2-benzo-thiazolamin (Talarozol) | 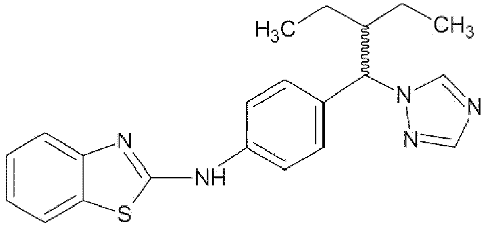 |

Die nachfolgenden Beispiele erläutern die Erfindung näher, ohne den Umfang der Erfindung zu beschränken.

### Beispiel 1

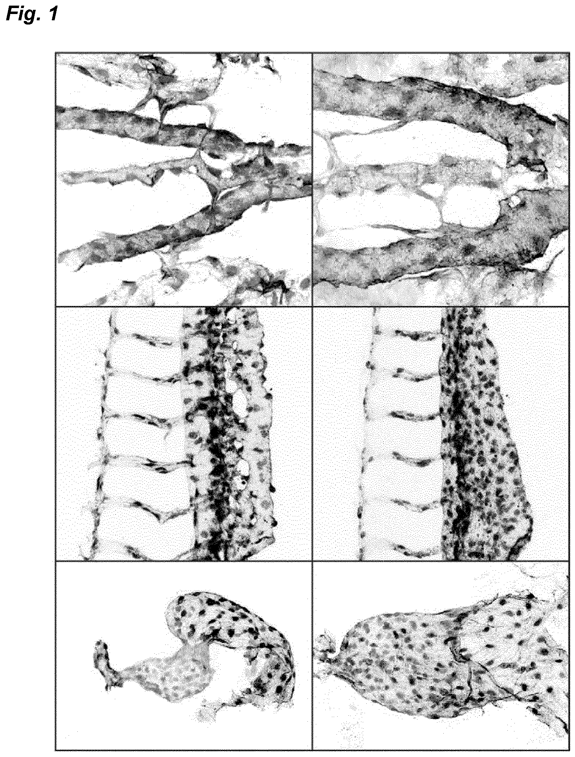

Die in Figur 1 dargestellten Aufnahmen zeigen einen Überblick eines Wildtyp-Zebrafischembryos und eines Zebrafischembryos mit einem *CCM2-*mutanten Phänotyp im Vergleich. Die drei Aufnahmen in der linken Spalte zeigen den Wildtyp-Zebrafischembryo, wohingegen die Aufnahmen in der rechten Spalte den *CCM2*-mutanten Phänotyp in einem Alter von zwei Tagen nach der Befruchtung zeigen. Des Weiteren zeigen die untersten Aufnahmen das Herz, die mittleren Aufnahmen den kaudalen Plexus und Intersegmentalgefäße und die oberen Aufnahmen die Aorta.

Die Aufnahmen des Zebrafischembryos des *CCM2*-mutanten Phänotyps zeigen in den entsprechenden Bereichen kardiovaskuläre Defekte im Vergleich zu dem entsprechenden Bereich des Wildtyp-Zebrafischembryos.

### Beispiel 2

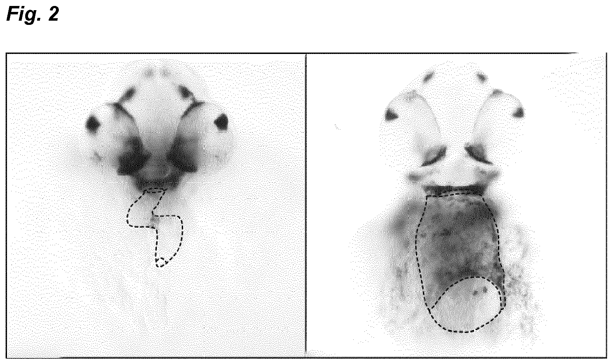

In der Figur 2 sind mikroskopische Aufnahmen dargestellt, welche die Expression der Retinaldehyd-Dehydrogenase 2 (*RALDH2*) im Zebrafischembryo zeigen.

Die linke Aufnahme zeigt die Expression der *RALDH2* im Herz eines Wildtyp-Zebrafisches und die rechte Aufnahme die Expression im Herz eines Zebrafisches mit *CCM2*-mutanten Phänotyp.

RALDH2, auch als Familienmitglied A2 bezeichnet, ist ein Familienmitglied der Aldehyd-Dehydrogenase 1 und wird im Menschen durch das Gen *ALDH1A2* kodiert. RALDH2 ist ein Enzym, welches die Synthese von Retinaldehyd zu Retinsäure katalysiert.

Die Aufnahmen in Figur 2 zeigen somit, dass die Expression des *RALDH2* Enzyms in dem Herzen eines *CCM2*-mutanten Zebrafisches, verglichen zur Expression in dem Herzen eines Wildtyp-Zebrafisches, stark erhöht ist. Es ist bekannt, dass der Mangel an Retinsäure zu einer Aktivierung von *RALDH2-*Expression führt.

Dies ist ein Hinweis darauf, dass die *CCM2*-Mutanten unter einem Retinsäuremangel leiden.

### Beispiel 3

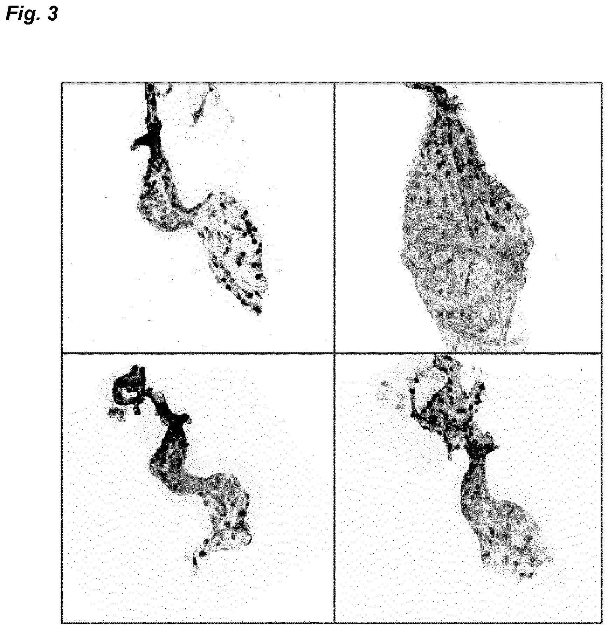

In Figur 3 sind mikroskopische Aufnahmen gezeigt, welche die Ergebnisse einer Behandlung mit Retinsäure in einem Wildtyp-Zebrafisch und einer *CCM-* Mutanten darstellen.

In einem Zeitraum von 16 - 51 Stunden nach der Befruchtung von Zebrafischeiern, wurde eine Gruppe von Zebrafischen mit einer 0,1 µM Retinsäurelösung und eine Gruppe von Zebrafischen mit einer 0,1-%igen Dimethylsulfoxid (DMSO)-Lösung behandelt. Die 0,1-%ige DMSO-Lösung wird als Kontrolle verwendet.

In Figur 3 zeigen die linken Aufnahmen beispielhaft die Herzen von behandelten Wildtyp-Zebrafischen und die rechten Aufnahmen die Herzen von behandelten *CCM2*-Mutanten. Die obere Reihe zeigt die Ergebnisse der Behandlung mit 0,1-%iger DMSO-Lösung und die untere Reihe die Ergebnisse der Behandlung mit einer 0,1 µM Retinsäurelösung.

Den histologischen Darstellungen in Figur 3 ist zu entnehmen, dass das mit DMSO als Kontrolle behandelte Herz der *CCM2*-Mutante im Vergleich zum entsprechenden Herzen des Wildtyp-Zebrafisches ballonartig vergrößert ist. Weiterhin zeigt Figur 3 eindeutig, dass die Behandlung mit Retinsäure bei dem Zebrafisch mit *CCM2*-mutanten Phänotyp dafür sorgt, dass die ballonartige Vergrößerung des Herzens sehr abgeschwächt ist und somit der *CCM2-*mutante Phänotyp insgesamt abgeschwächt ausgeprägt ist und dem Phänotyp des Wildtyps sehr nahe kommt.

Demnach zeigen die Beispiele, den starken pharmakologischen unterdrückenden Effekt einer Verbindung der Formel I, insbesondere von Retinsäure, auf die Ausbildung der kardiovaskulären Phänotypen in *CCM-* Mutanten im Zebrafischmodell.

### Beispiel 4

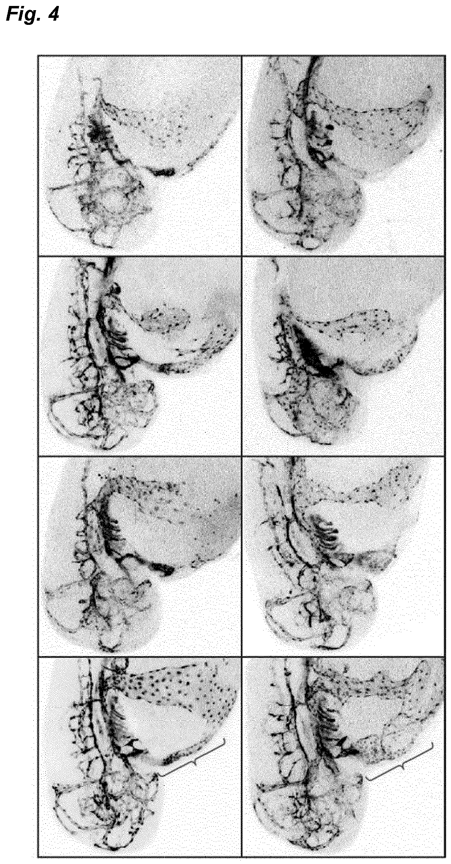

In Figur 4 sind beispielhaft die Effekte anderer Wirkstoffe auf die Ausprägung von behandelten *CCM2*-mutanten Herzen im Vergleich zum Herzen des Wildtyp-Zebrafisches und einer *CCM2*-Mutante nach Behandlung mit einer 0,1 %igen DMSO Lösung abgebildet (Region des Herzens ist durch schwarze Klammern markiert).

In einem Zeitraum von 16 - 51 Stunden nach der Befruchtung von Zebrafischeiern fand die Behandlung mit unterschiedlichen Wirkstoffen statt. Die Behandlung erfolgte mit 0,1 % DMSO (unterste Reihe), 1 µM Retinal (zweite Reihe von unten), 1 µM Retinol (zweite Reihe von oben)und 10 µM Talarozole (oberste Reihe). Die linke Spalte zeigt die Ergebnisse nach der Behandlung im Herzen von Wildtyp-Zebrafischen mit den unterschiedlichen Wirkstoffen und die rechte Spalte zeigt die Ergebnisse im Herzen von *CCM2-*Mutanten nach der Behandlung mit den unterschiedlichen Wirkstoffen.

Diesen Teilabbildungen ist zu entnehmen, dass das mit DMSO als Kontrolle behandelte Herz der *CCM2*-Mutante im Vergleich zum entsprechenden Herzen des Wildtyp-Zebrafisches ballonartig vergrößert ist. Weiterhin zeigt Figur 4 eindeutig, dass die Behandlung mit den aufgeführten Wirkstoffen bei dem Zebrafisch mit *CCM2*-mutanten Phänotyp dafür sorgt, dass die ballonartige Vergrößerung des Herzens sehr abgeschwächt ist und somit der *CCM2-*mutante Phänotyp insgesamt abgeschwächt ausgeprägt ist und dem Phänotyp des Wildtyps sehr nahe kommt.

Diese Beispiele belegen die starke pharmakologische Unterdrückung des *CCM2*-mutanten Herzphänotypen im Zebrafischmodell.

## Patentansprüche

1. Verbindung der allgemeinen Formel I 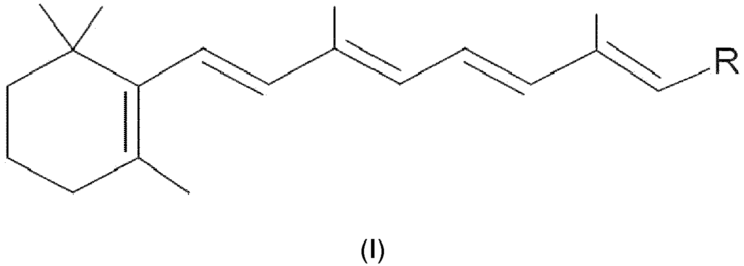 wobei R ausgewählt ist aus der Gruppe bestehend aus -CHO, -COOH, -CH₂OH und 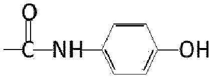 sowie deren Stereoisomere, Enantiomere oder Diastereomere, deren physiologisch annehmbaren Salze sowie Mischungen der genannten Verbindungen zur Anwendung bei der Behandlung einer Blutgefäßerkrankung.

2. Verbindung zur Anwendung bei der Behandlung einer Blutgefäßerkrankung, gemäß Anspruch 1, **dadurch gekennzeichnet, dass** die Blutgefäßerkrankung ausgewählt ist aus zerebralen kavernösen Malformationen, hämorrhagischen Schlaganfällen, hereditären hämorrhagischen Teleangiektasien oder arterio-venösen Malformationen.

3. Verbindung zur Anwendung bei der Behandlung einer Blutgefäßerkrankung, gemäß einem der Ansprüche 1 oder 2, **dadurch gekennzeichnet, dass** die Verbindung in einer oral applizierbaren Form vorliegt, wobei die oral applizierbare Form eine Kapsel, eine Tablette, eine Suspension, eine Lösung oder ein Dragee ist oder dass die Verbindung in einer intravenös applizierbaren Form vorliegt, wobei die intravenös applizierbare Form eine Suspension oder eine Lösung ist oder dass die Verbindung in einer subkutan applizierbaren Form vorliegt, wobei die subkutan applizierbare Form eine Suspension, eine Lösung oder ein Pellet ist.

4. Verbindung zur Anwendung bei der Behandlung einer Blutgefäßerkrankung, gemäß einem der Ansprüche 1 bis 3, **dadurch gekennzeichnet, dass** die Verabreichung der Verbindung gemäß einem Dosierschema erfolgt, wobei die Dosierung der Verbindung in Abhängigkeit von Alter, Geschlecht und Gewicht eines Patienten, der eine Blutgefäßerkrankung aufweist, sowie von Art und Schwere der Blutgefäßerkrankung festgelegt wird und die Dauer der Behandlung mindestens einen Tag beträgt.

5. Pharmazeutische Zusammensetzung zur Anwendung bei der Behandlung einer Blutgefäßerkrankung, umfassend eine Verbindung gemäß einem der Ansprüche 1 bis 4 sowie die physiologisch annehmbaren Salze und Stereoisomere, Enantiomere, Diastereomere oder Mischungen daraus und pharmazeutisch annehmbare Hilfsstoffe und/oder Trägerstoffe in Kombination mit einer oder mehreren Substanzen, ausgewählt aus der Gruppe bestehend aus Indirubin-3-monoxim, Indirubin, Vitamin D, Vitamin D3, Rapamycin, Statinen, Sulindac und seinen Metaboliten, RhoA, Rho-Kinase-Inhibitoren, Gefäßwachstumsfaktor-Inhibitoren. Inhibitoren der Polymerase-Kettenreaktion, Mek/Erk-Inhibitoren, Propranolol, Propranolol-Hydrochlorid und Antioxidantien.

6. Pharmazeutische Zusammensetzung zur Anwendung bei der Behandlung einer Blutgefäßerkrankung, gemäß Anspruch 5, **dadurch gekennzeichnet, dass** die Blutgefäßerkrankung ausgewählt ist aus zerebralen kavernösen Malformationen, hämorrhagischen Schlaganfällen, hereditären hämorrhagischen Teleangiektasien oder arterio-venösen Malformationen.

7. Pharmazeutische Zusammensetzung zur Anwendung bei der Behandlung einer Blutgefäßerkrankung, gemäß einem der Ansprüche 5 oder 6, **dadurch gekennzeichnet, dass** die pharmazeutische Zusammensetzung in einer oral applizierbaren Form vorliegt, wobei die oral applizierbare Form eine Kapsel, eine Tablette, eine Suspension, eine Lösung oder ein Dragee ist oder dass die pharmazeutische Zusammensetzung in einer intravenös applizierbaren Form vorliegt, wobei die intravenös applizierbare Form eine Suspension oder eine Lösung ist oder dass die pharmazeutische Zusammensetzung in einer subkutan applizierbaren Form vorliegt, wobei die subkutan applizierbare Form eine Suspension, eine Lösung oder ein Pellet ist.

8. Pharmazeutische Zusammensetzung zur Anwendung bei der Behandlung einer Blutgefäßerkrankung, gemäß einem der Ansprüche 5 bis 7, **dadurch gekennzeichnet, dass** die Verabreichung der pharmazeutischen Zusammensetzung gemäß einem Dosierschema erfolgt, wobei die Dosierung in Abhängigkeit von Alter, Geschlecht und Gewicht eines Patienten, der eine Blutgefäßerkrankung aufweist, sowie von Art und Schwere der Blutgefäßerkrankung festgelegt wird und die Dauer der Behandlung mindestens einen Tag beträgt.

9. Verfahren zur Behandlung einer Blutgefäßerkrankung, wobei man einem Patienten, der mindestens eine vaskuläre Läsion aufweist oder das Risiko aufweist, eine vaskuläre Läsion zu entwickeln, eine therapeutisch wirksame Menge einer Verbindung gemäß einem der Ansprüche 1 bis 4 sowie die physiologisch verträglichen Salze und Stereoisomere, Enantiomere, Diastereomere oder Mischungen daraus verabreicht.

10. Verfahren gemäß Anspruch 9, **dadurch gekennzeichnet, dass** die Blutgefäßerkrankung ausgewählt ist aus zerebralen kavernösen Malformationen, hämorrhagischen Schlaganfällen, hereditären hämorrhagischen Teleangiektasien oder arterio-venösen Malformationen.

11. Verfahren gemäß Anspruch 9 oder 10, **dadurch gekennzeichnet, dass** man vor der Verabreichung sowie nach der Verabreichung der therapeutischen Menge der Verbindung, die Anzahl von vaskulären Läsionen und/oder Größe von mindestens einer vaskulären Läsion sowie das Stadium der Erkrankung erfasst.

12. Verfahren gemäß Anspruch 11, **dadurch gekennzeichnet, dass** man die Erfassung der Anzahl von vaskulären Läsionen und/oder die Größe der mindestens einen vaskulären Läsion mittels einer Magnetresonanztomographie durchführt.

13. Verfahren, gemäß einem der Ansprüche 9 bis 12, **dadurch gekennzeichnet, dass** die Verbindung oral in Form einer Kapsel, einer Tablette, einer Suspension, einer Lösung oder eines Dragees oder intravenös in Form einer Suspension oder Lösung oder subkutan in Form einer Suspension, einer Lösung oder eines Pellets verabreicht wird.

14. Verfahren, gemäß einem der Ansprüche 9 bis 13, **dadurch gekennzeichnet, dass** das Verfahren einen Schritt umfasst, in dem ein Patient identifiziert wird, der mindestens eine vaskuläre Läsion aufweist oder das Risiko aufweist, mindestens eine vaskuläre Läsion zu entwickeln.

15. Verfahren, gemäß Anspruch 14, **dadurch gekennzeichnet, dass** zur Identifizierung eines Patienten, der eine zerebrale kavernöse Malformation aufweist oder das Risiko aufweist eine zerebrale kavernöse Malformation zu entwickeln, eine Mutation in zumindest einem Gen des Patienten identifiziert wird, welches mit der zerebralen kavernösen Malformation assoziiert ist, wobei das Gen ausgewählt ist aus *CCM2, KRIT1* oder *PDCD10.*
